# EUROPÄISCHE PATENTSCHRIFT

(11) **EP 1 108 469 B1**
(45) Veröffentlichungstag und Bekanntmachung des Hinweises auf die Patenterteilung: **29.12.2004**
(21) Anmeldenummer: 00126921.6
(22) Anmeldetag: 08.12.2000
(51) Int. Cl.: B01J 25/00, C07C 209/48

(54) **Modifikation eines Hydrierungskatalysators**
Hydrogenation catalyst activation
Activation d'un catalyseur d'hydrogénation

(30) Priorität: 16.12.1999 EP 99125081; 28.09.2000 EP 00121068
(43) Veröffentlichungstag der Anmeldung: 20.06.2001
(73) Patentinhaber: DSM IP Assets B.V., 6411 TE Heerlen (NL)
(72) Erfinder: Degischer, Oliver Gerald, 8045 Zürich (CH); Roessler, Felix, 4303 Kaiseraugst (CH)
(74) Vertreter: Schwander, Kuno Josef, Dr.

(56) Entgegenhaltungen:
- GB-A- 1 206 981
- US-A- 3 953 367
- DATABASE WPI Section Ch, Week 197810 Derwent Publications Ltd., London, GB; Class J04, AN 1978-18506A XP002162745 & JP 53 008288 A (JGC CORP), 25. Januar 1978 (1978-01-25)

## Beschreibung

Die vorliegende Erfindung betrifft ein Verfahren zur Modifikation eines Hydrierungskatalysators vom Typ Raney Nickel, Raney Cobalt, Nickel auf Träger oder Cobalt auf Träger. Infolge des Verfahrens, bei dem man einen derartigen Katalysator mit Kohlenmonoxid, Kohlendioxid, Formaldehyd, einem niederen aliphatischen Aldehyd, einem aromatischen Aldehyd, einem aliphatischen Keton, einem aromatischen Keton, einem gemischt aliphatisch/aromatischen Keton, Glyoxal, Pyruvaldehyd oder Glyoxylsäure behandelt, wird beim Einsatz des so modifizierten Katalysators in der Hydrierung eines Nitrils zum entsprechenden Amin die Selektivität gesteigert. Die gesteigerte Selektivität begünstigt wesentlich den Anteil des primären Amins gegenüber dem unerwünschten sekundären Amin in dem Hydrierungsprodukt im Vergleich zum Fall, bei dem der entsprechende unmodifizierte Katalysator eingesetzt wird. Die Art der Modifikation und die daraus resultierende gesteigerte Selektivität des Katalysators sind überraschend. Demzufolge betrifft die vorliegende Erfindung als weiteren Aspekt das Verfahren zur Hydrierung eines Nitrils zum entsprechenden primären Amin unter Verwendung eines so modifizierten Katalysators.

Das erfindungsgemässe Verfahren zur Modifikation eines Hydrierungskatalysators vom Typ Raney Nickel, Raney Cobalt, Nickel auf Träger oder Cobalt auf Träger ist insbesondere dadurch gekennzeichnet, dass man den Hydrierungskatalysator bei Temperaturen von etwa 0°C bis etwa 120°C mit Kohlenmonoxid, Kohlendioxid, Formaldehyd, einem niederen aliphatischen Aldehyd, einem aromatischen Aldehyd, einem aliphatischen Keton, einem aromatischen Keton, einem gemischt aliphatisch/aromatischen Keton, Glyoxal, Pyruvaldehyd oder Glyoxylsäure als Modifikationsmittel in einem aus Wasser oder einem organischen Lösungsmittel bestehenden flüssigen Dispersionsmittel für die Dauer von etwa 15 Minuten bis etwa 24 Stunden behandelt.

Im Falle von Formaldehyd als Modifikationsmittel kann dieser auch in Form von Metaldehyd oder Paraformaldehyd verwendet werden. Geeigneterweise wird Formaldehyd in Form seiner wässrigen Lösung, d.h als Formalin, eingesetzt, wobei dann Wasser zumindest einen Teil des flüssigen Dispersionsmittels bildet.

Bei dem als Modifikationsmittel verwendbaren "niederen aliphatischen Aldehyd" handelt es sich insbesondere um einen Aldehyd der Formel R¹CHO, in welcher R¹ eine gegebenenfalls mit Hydroxy substituierte Alkylgruppe mit 1 bis 5 Kohlenstoffatomen bedeutet. Je nach Anzahl der Kohlenstoffatome kann die Alkylgruppe geradkettig oder verzweigt sein. Im Falle, dass die Alkylgruppe mit Hydroxy substituiert ist, können einer oder mehrere Hydroxysubstituenten vorhanden sein. Vorzugsweise wird als derartiges Modifikationsmittel Acetaldehyd verwendet.

Bei dem als Modifikationsmittel verwendbaren "aromatischen Aldehyd" handelt es sich insbesondere um einen Aldehyd der Formel R²CHO, in welcher R² eine Aryl- oder Heteroarylgruppe bedeutet. Der Ausdruck "Aryl" umfasst nicht nur die üblichen unsubstituierten Arylgruppen, d.h. Phenyl und Naphthyl, sondern auch die entsprechenden substituierten Phenyl- bzw. Naphthylgruppen. Als Substituenten kommen beispielsweise Halogenatome und C₁₋₄-Alkyl-, Hydroxy-, C₁₋₄-Alkoxy-, Amino-, Carbamoyl- und Phenylgruppen in Frage, wobei jeweils ein oder mehrere Substituenten vorhanden sein können. Unter dem Ausdruck "Halogen" ist Fluor, Chlor, Brom oder Jod zu verstehen. Eine Alkyl- oder Alkoxygruppe kann je nach Anzahl der Kohlenstoffatome geradkettig oder verzweigt sein. Im Falle von mehreren Substituenten können die Substituenten gleich oder verschieden sein. Es können normalerweise nicht mehr als 5 (für Phenyl) bzw. 7 (für Naphthyl) Halogenatome, 3 Alkylgruppen, 2 Hydroxygruppen, 3 Alkoxygruppen 2 Aminogruppen, 2 Carbamoylgruppen oder eine Phenylgruppe vorhanden sein. Der Ausdruck "Heteroaryl" umfasst seinerseits Heteroarylgruppen, welche eins oder mehrere Heteroatome im Ring aufweisen, wie insbesondere Stickstoff-, Sauerstoff- und/oder Schwefelatome; Beispiele solcher Heteroarylgruppen sind Pyridyl und Pyrimidinyl. Vorzugsweise wird als aromatischer Aldehyd in der Rolle des Modifikationsmittels Benzaldehyd oder Anisaldehyd verwendet.

Bei dem als Modifikationsmittel verwendbaren "aliphatischer, aromatischer oder gemischt aliphatisch/aromatischer Keton" handelt es sich insbesondere um einen Keton der Formel R³COR⁴, in welcher R³ bzw. R⁴ unabhängig von dem anderen Symbol eine Alkyl-, Aryl- oder Heteroarylgruppe bedeutet. Der Ausdruck "Alkyl", "Aryl" bzw. "Heteroaryl" ist jeweils wie oben im Zusammenhang mit den Definitionen von R¹ und R² zu verstehen.Vorzugsweise wird als derartiges Modifikationsmittel Aceton verwendet.

In einer Ausführungsform des erfindungsgemässen Modifikationsverfahrens ist das Modifikationsmittel Kohlenmonoxid, Formaldehyd oder ein niederer aliphatischer Aldehyd

Die im erfindungsgemässen Modifikationsverfahren modifizierbaren Hydrierungskatalysatoren vom Typ Raney Nickel und Raney Cobalt sind dem Fachmann bestens bekannt und im Handel leicht erhältlich, so dass sich eine weitere Erläuterung erübrigt. Das Gleiche gilt für den ebenfalls im Verfahren modifizierbaren Katalysator vom Typ Nickel auf Träger oder Cobalt auf Trager. Als Träger in diesen beiden Fällen kommen beispielsweise Silika, Titanoxid, Aluminiumoxid, Kieselgur und Aktivkohle in Frage.

Geeignete organische Lösungsmittel, in welchen neben Wasser die Modifikation erfindungsgemäss erfolgen kann, sind insbesondere aliphatische Kohlenwasserstoffe, z.B. Pentan und Hexan, aromatische Kohlenwasserstoffe, z.B. Benzen und Toluen; Alkanole, z.B. Methanol, Ethanol und Propanol; aliphatische und cyclische Ether, z.B. Diethylether bzw. Tetrahydrofuran und Dioxan; sowie Heteroaromaten, z.B. Pyridin. Das Dispersionsmittel kann aus Wasser allein oder einem einzigen organischen Lösungsmittel bestehen, oder aus zwei oder mehreren solchen Flüssigkeiten. Beispielsweise kann ein wässriges Alkanol, z.B. wässriges Ethanol, als flüssiges Dispersionsmittel verwendet werden. Im Allgemeinen wird zur Durchführung des Verfahrens der Hydrierungskatalysator in Wasser und/oder einem solchen organischen Lösungsmittel dispergiert, da eine Auflösung des Katalysators im Hinblick auf dessen Natur nicht stattfindet. Hingegen muss sich das Modifikationsmittel im Dispersionsmittel zumindest teilweise auflösen, wie dies unten näher erläutert wird.

Bezogen auf die Menge des eingesetzten zu modifizierenden Hydrierungskatalysators beträgt die Menge des eingesetzten Modifikationsmittels in den verschiedenen Fällen wie folgt:

| Modifikationsmittel | Zweckmässigerweise | Vorzugsweise |
|---|---|---|
| Kohlenmonoxid | 0,5-5 Gewichtsprozent (Gew.%) | 1-2 Gew.% |
| Kohlendioxid | 1-30 Gew.% | 5-15 Gew.% |
| Formaldehyd, Glyoxal, Pyruvaldehyd, Glyoxylsäure | 0,25-50 Gew.% | 1-15 Gew.% |
| Niederer aliphatischer Aldehyd | 0,25-50 Gew.% | 1-15 Gew.% |
| Aromatischer Aldehyd | 0,25-50 Gew.% | 1-15 Gew.% |
| aliphatischer, aromatischer oder gemischt aliphatisch/ aromatischer Keton | 0,25-50 Gew.% | 5-20 Gew.% |

Die eingesetzte Menge an Dispersionsmittel muss den Hydrierungskatalysator dispergieren, damit das Modifikationsmittel seine Wirkung auf den Katalysator möglichst viel entfalten kann. Die geeignete Menge hängt von vielen Faktoren ab, wie beispielsweise dem Reaktordesign und der Drehzahl eines verwendeten Rührers, und kann durch entsprechenden Versuche vom Fachmann problemlos etabliert werden.

Der Temperaturbereich, in welchem das erfindungsgemässe Modifikationsverfahren durchgeführt wird, beträgt im Allgemeinen etwa 0°C bis etwa 120°C. Vorzugsweise erfolgt das Verfahren bei Raumtemperatur.

Je nach verwendetem Modifikationsmittel und flüssigem Dispersionsmittel, nach eingesetzter Menge des Modifikationsmittels sowie nach der Temperatur, bei der das Modifikationsverfahren durchgeführt wird, kann das Modifikationsmittel in vollständig gelöster oder in teilweise gelöster Form wirken. Formaldehyd wird beispielsweise üblicherweise in gelöster Form eingesetzt, und zwar in wässriger Lösung (als Formalin), Paraformaldehyd hingegen eher in fast ungelöster (fester) Form. Während der Modifikation muss durch ausreichende Durchmischung dafür gesorgt werden, dass eine möglichst homogene Verteilung des Modifikationsmittels im Dispersionsmittel gewährleistet ist; dies ist insbesondere wichtig, wenn das Modifikationsmittel, wie beispielsweise im Falle von Kohlenmonoxid oder Paraformaldehyd, eine schlechte oder nur beschränkte Löslichkeit im Dispersionsmittel aufweist. Zu diesem Zwecke wird geeigneterweise während des Modifikationsverfahrens gerührt oder geschüttelt, um die homogene Verteilung zu fördern.

Im weiteren ist es von Vorteil, durch Einsatz eines Inertgases, z.B. Stickstoff oder Argon, den Kontakt des Hydrierungskatalysators mit Sauerstoff möglichst zu vermeiden. Auf diese Weise wird eine unerwünschte Desaktivierung des Katalysators minimalisiert.

Wie oben erwähnt, erreicht man beim Einsatz des erfindungsgemäss modifizierten Hydrierungskatalysators in einer Hydrierung eines Nitrils eine überraschend gute Selektivität zugunsten des entsprechenden primären Amins; es werden weniger Nebenprodukte, insbesondere weniger sekundäres und tertiäres Amin, erzeugt, verglichen mit dem Fall, bei dem der unmodifizierte Hydrierungskatalysator eingesetzt wird. Die Selektivitätssteigerung wird bei gleichbleibender Aktivität des Hydrierungskatalysators erreicht. Somit können Hydrierungen von Nitrilen insgesamt wirtschaftlicher durchgeführt werden.

Ein weiterer Vorteil des erfindungsgemäss modifizierten Hydrierungskatalysators besteht darin, dass er nach der Modifikation erfahrungsgemäss lange Zeit unter Wasser gelagert werden kann, ohne seine Aktivität und Selektivität zu verlieren, und zwar mindestens 30 Tage. Der Einsatz des Katalysators in einer Hydrierung muss deshalb nicht unmittelbar nach dessen Modifikation vorgenommen werden.

Bei dem weiteren erfindungsgemässen Verfahren handelt es sich um die vorteilhafte Anwendung des erfindungsgemäss modifizierten Hydrierungskatalysators, und zwar um ein Verfahren zur katalytischen Hydrierung eines Nitrils zum entsprechenden primären Amin, das dadurch gekennzeichnet ist, dass man als Hydrierungskatalysator einen verwendet, welcher gemäss dem oben definierten Modifikationsverfahren erhalten wurde.

Der in diesem Hydrierungsverfahren zu verwendende erfindungsgemäss modifizierte Hydrierungskatalysator kann nach Wunsch entweder erst nach Isolierung aus dem Gemisch, welches nach Beendung des Modifikationsverfahrens erhalten wird, oder unmittelbar und ohne Isolierung in diesem Gemisch eingesetzt werden. Im ersten Fall kann der modifizierte Katalysator durch Sedimentation oder Filtration und Aufschlämmung mit frischem Dispersionsmittel bzw. Spülung des Filterkuchens gewaschen werden.Es kann dasselbe Lösungsmittel für das Modifikationsverfahren verwendet werden wie jenes, das üblicherweise für die anschliessende Hydrierung benutzt wird, so dass im zweiten Fall eine zwischenstufige Ergänzung der Lösungsmittelmenge eventuall benötigt wird. In diesem Fall ist aber sicherzustellen, dass das Modifikationsmittel aufgebraucht ist, d.h. nicht mehr nachweisbar ist, um in der anschliessenden Hydrierung Nebenreaktionen zu vermeiden.

Im Prinzip können alle Nitrile durch das erfindungsgemässe Hydrierungsverfahren selektiver zu den entsprechenden primären Aminen hydriert werden. Insbesondere werden aliphatische Nitrile, z.B. Alkyl- und Alkenylnitrile, Arylnitrile sowie Heteroarylnitrile hydriert. Der Ausdruck "Aryl" umfasst nicht nur die üblichen unsubstituierten Arylgruppen, d.h. Phenyl und Naphthyl, sondern auch die entsprechenden substituierten Phenyl- bzw. Naphthylgruppen, wie diese oben im Zusammenhang mit den Definitionen von R², R³ und R⁴ näher erläutert sind. Ebenfalls sind unter dem Ausdruck "Heteroaryl" diejenigen derartigen Gruppen zu verstehen, die oben im Zusammenhang mit den Definitionen von R², R³ und R⁴ näher erläutert sind.

Mit Ausnahme der erfindungsgemässen Anwendung des modifizierten Hydrierungskatalysators kann das Hydrierungsverfahren unter an sich bekannten Hydrierungsbedingungen, insbesondere in Bezug auf Lösungsmittel, Temperatur, Druck, Menge des Hydrierungskatalysators sowie Dauer der Hydrierungsreaktion, durchgeführt werden. Um möglichst selektiv zum jeweiligen primären Amin zu gelangen, wird allerdings vorzugsweise in reinem Ammoniak oder in zu diesem Zweck an sich bekannten Gemischen von Ammoniak und einem organischen Lösungsmittel hydriert. In solchen Gemischen eignen sich als organische Lösungsmittel diejenigen, die oben im Zusammenhang mit dem Dispersionsmittel erwähnt sind.

Auch die Isolierung und die Reinigung des jeweiligen Hydrierungsproduktes können nach an sich bekannten Methoden durchgeführt werden.

Die nachfolgenden Beispiele veranschaulichen die erfindungsgemässen Verfahren (Modifikations- bzw. Hydrierungsverfahren); in diesen Beispielen beziehen sich die Konzentrationsangaben auf das Gewicht des jeweils erfindungsgemäss modifizierten Hydrierungskatalysators und die Selektivität auf Hydrierungen mit 100%igem Umsatz bezüglich des Nitrils und eventueller Zwischenprodukte, die während der Reaktion zwischenzeitlich entstehen können.

### Beispiel 1

### Formaldehyd als Modifikationsmittel

40 g Pynitril (4-Amino-5-cyano-2-methylpyrimidin) wurden in Gegenwart von 5 g kommerziell erhältlichem Raney Nickel, 1,475 l Methanol und 300 g Ammoniak bei einer Temperatur von 110°C und 40 bar (4 MPa) Gesamtdruck hydriert. Nach 5 Stunden wurde eine Ausbeute von 96,4% primäres Amin (4-Amino-5-aminomethyl-2-methylpyrimidin) erreicht, wobei auch noch sekundäres Amin in 1,9%iger Ausbeute erzeugt wurde.

Wird der Raney Nickel (5 g) vor der Hydrierung mit 12 g Wasser und 0,36 g 35%iger wässriger Formaldehydlösung 30 Minuten bei Raumtemperatur (25°C) unter Stickstoff oder Argon gerührt, danach abdekantiert und dreimal mit je 50 ml entionisiertem Wasser nachgewaschen, erreicht man unter Verwendung des so modifizierten Katalysators nach 5 Stunden eine Selektivität von 99,6% (Anteil sekundäres Amin < 0,1%). Wird der modifizierte Katalysator erst nach 30-tägiger Lagerung unter Wasser eingesetzt, erreicht man eine Selektivität von 99,7% (Anteil sekundäres Amin < 0,1%).

### Beispiel 2

### Kohlenmonoxid als Modifikationsmittel

40 g Pynitril wurden in Gegenwart von 5 g kommerziell erhältlichem Raney Nickel, 1,475 l Methanol und 300 g Ammoniak bei einer Temperatur von 110°C und 40 bar (4 MPa) Gesamtdruck hydriert. Nach 5 Stunden wurde eine Ausbeute von 96,4% primäres Amin erreicht, wobei auch noch sekundäres Amin in 1,9%iger Ausbeute erzeugt wurde.

Wird der Raney Nickel (5 g) vor der Hydrierung mit 12 g Wasser und 50 ml Kohlenmonoxid eine Stunde bei Raumtemperatur (25°C) unter Stickstoff oder Argon gerührt, danach abdekantiert und dreimal je 50 ml entionisiertem Wasser nachgewaschen, erreicht man unter Verwendung des so modifizierten Katalysators nach 5 Stunden eine Selektivität von 98,8% (Anteil sekundäres Amin < 0,1 %).

### Beispiel 3

### Acetaldehyd als Modifikationsmittel

40 g Pynitril wurden in Gegenwart von 6 g kommerziell erhältlichem Raney Nickel, 1,475 l Methanol und 300 g Ammoniak bei einer Temperatur von 110°C und 40 bar (4 MPa) Gesamtdruck hydriert. Nach 5 Stunden wurde eine Ausbeute von 96,4% primäres Amin erreicht, wobei auch noch sekundäres Amin in 1,9%iger Ausbeute erzeugt wurde.

Wird der Raney Nickel (6 g) vor der Hydrierung mit 15 g Wasser und 0,2 g Acetaldehyd eine Stunde bei 60°C unter Stickstoff oder Argon gerührt, danach abdekantiert und dreimal mit je 50 ml entionisiertem Wasser nachgewaschen, erreicht man unter Verwendung des so modifizierten Katalysators nach 5 Stunden eine Selektivität von 97,3% (Anteil sekundäres Amin < 0,1%).

### Beispiel 4

### Formaldehyd als Modifikationsmittel

40 g Pynitril wurden in Gegenwart von 10 g kommerziell erhältlichem Raney Cobalt, 1,475 l Methanol und 300 g Ammoniak bei einer Temperatur von 110°C und 40 bar (4 MPa) Gesamtdruck hydriert. Nach 5 Stunden wurde eine Ausbeute von 96,8% primäres Amin erreicht, wobei auch noch sekundäres Amin in 2,2%iger Ausbeute erzeugt wurde.

Wird der Raney Cobalt (10 g) vor der Hydrierung mit 24 g Wasser und 0,72 g 35 %iger Formaldehydlösung eine Stunde bei Raumtemperatur (25°C) unter Stickstoff oder Argon gerührt, danach abdekantiert und dreimal mit je 50 ml entionisiertem Wasser nachgewaschen, erreicht man unter Verwendung des so modifizierten Katalysators nach 5 Stunden eine Selektivität von 99,1% (Anteil sekundäres Amin < 0,1%).

### Beispiel 5

### Formaldehyd als Modifikationsmittel

40 g Pynitril wurden in Gegenwart von 8 g kommerziell erhältlichem Nickel auf Silika 1,475 l Methanol und 300 g Ammoniak bei einer Temperatur von 110°C und 40 bar (4 MPa) Gesamtdruck hydriert. Nach 5 Stunden wurde eine Ausbeute von 96,8% primäres Amin erreicht, wobei auch noch sekundäres Amin in 1,9%iger Ausbeute erzeugt wurde.

Wird der Nickel auf Silika (8 g) vor der Hydrierung mit 20 g Wasser und 0,57 g 35%iger Formaldehydlösung eine Stunde bei Raumtemperatur (25°C) unter Stickstoff oder Argon gerührt, danach abdekantiert und dreimal mit je 50 ml entionisiertem Wasser nachgewaschen, erreicht man unter Verwendung des so modifizierten Katalysators nach 5 Stunden eine Selektivität von 98,1% (Anteil sekundäres Amin < 0,1%).

### Beispiel 6

### Formaldehyd als Modifikationsmittel

100 g Benzonitril wurden in Gegenwart von 5,7 g kommerziell erhältichem Raney Nickel und 2 l Methanol bei einer Temperatur von 100°C und 40 bar (4 MPa) Gesamtdruck hydriert. Nach 3 Stunden wurde eine Ausbeute von 70,0% primäres Amin (Benzylamin) erreicht, wobei auch noch Dibenzylamin in 29,8%iger Ausbeute erzeugt wurde.

Wird der Raney Nickel (5,7 g) vor der Hydrierung mit 13,7 g Wasser und 0,41 g 35%iger wässriger Formaldehydlösung eine Stunde bei Raumtemperatur (25°C) unter Stickstoff oder Argon gerührt, danach abdekantiert und dreimal mit 50 ml entionisiertem Wasser nachgewaschen, erreicht man unter Verwendung des so modifizierten Katalysators nach 3 Stunden eine Selektivität von 83,6% (Anteil Dibenzylamin 12,6%).

### Beispiel 7

### Formaldehyd als Modifikationsmittel

100 g Benzonitril wurden in Gegenwart von 5,7 g kommerziell erhältichem Raney Nickel, 2 l Methanol und 15 g Ammoniak bei einer Temperatur von 100°C und 40 bar (4 MPa) Gesamtdruck hydriert. Nach 3 Stunden wurde eine Ausbeute von 84,0% primäres Amin (Benzylamin) erreicht, wobei auch noch Dibenzylamin in 15,4%iger Ausbeute erzeugt wurde.

Wird der Raney Nickel (5,7 g) vor der Hydrierung mit 13,7 g Wasser und 0,41 g 35%iger wässriger Formaldehydlösung eine Stunde bei Raumtemperatur (25°C) unter Stickstoff oder Argon gerührt, danach abdekantiert und dreimal mit 50 ml entionisiertem Wasser nachgewaschen, erreicht man unter Verwendung des so modifizierten Katalysators nach 3 Stunden eine Selektivität von 94,1% (Anteil Dibenzylamin 5,3%).

### Beispiel 8

### Formaldehyd als Modifikationsmittel

40,6 g Pyridin-3-carbonitril wurden in Gegenwart von 5,3 g kommerziell erhältichem Raney Nickel, 2 1 Methanol und 31 g Ammoniak bei einer Temperatur von 100°C und 40 bar (4 MPa) Gesamtdruck hydriert. Nach 5 Stunden wurde eine Ausbeute von 78,1% primäres Amin (3-Aminomethyl-pyridin) erreicht, wobei auch noch sekundäres Amin in 17,7%iger Ausbeute erzeugt wurde.

Wird der Raney Nickel (5,3 g) vor der Hydrierung mit 12,7 g Wasser und 0,38 g 35%iger wässriger Formaldehydlösung eine Stunde bei Raumtemperatur (25°C) unter Stickstoff oder Argon gerührt, danach abdekantiert und dreimal mit 50 ml entionisiertem Wasser nachgewaschen, erreicht man unter Verwendung des so modifizierten Katalysators nach 3 Stunden eine Selektivität von 87,0% (Anteil sekundäres Amin 8,9%).

### Beispiel 9

### Benzaldehyd als Modifikationsmittel

100 g Benzonitril wurden in Gegenwart von 5,7 g kommerziell erhältichem Raney Nickel, 2 l Methanol und 15 g Ammoniak bei einer Temperatur von 100°C und 40 bar (4 MPa) Gesamtdruck hydriert. Nach 3 Stunden wurde eine Ausbeute von 84,0% primäres Amin (Benzylamin) erreicht, wobei auch noch Dibenzylamin in 15,4%iger Ausbeute erzeugt wurde.

Wird der Raney Nickel (5,9 g) vor der Hydrierung mit 14,1 g Wasser und 0,37 g Benzaldehyd eine Stunde bei Raumtemperatur (25°C) unter Stickstoff oder Argon gerührt, danach abdekantiert und dreimal mit 50 ml entionisiertem Wasser nachgewaschen, erreicht man unter Verwendung des so modifizierten Katalysators nach 3 Stunden eine Selektivität von 94,2% (Anteil Dibenzylamin 4,8%).

### Beispiel 10

### Kohlendioxid als Modifikationsmittel

100 g Benzonitril wurden in Gegenwart von 5,7 g kommerziell erhältichem Raney Nickel, 2 l Methanol und 15 g Ammoniak bei einer Temperatur von 100°C und 40 bar (4 MPa) Gesamtdruck hydriert. Nach 3 Stunden wurde eine Ausbeute von 84,0% primäres Amin (Benzylamin) erreicht, wobei auch noch Dibenzylamin in 15,4%iger Ausbeute erzeugt wurde.

Wird der Raney Nickel (6,0 g) vor der Hydrierung mit 15,0 g Wasser und 200 ml Kohlendioxid 24 Stunden bei Raumtemperatur (25°C) unter Stickstoff oder Argon gerührt, danach abdekantiert, erreicht man unter Verwendung des so modifizierten Katalysators nach 3 Stunden eine Selektivität von 91,8% (Anteil Dibenzylamin 8,1%).

### Beispiel 11

### Formaldehyd als Modifikationsmittel

100 ml Valeronitril wurden in Gegenwart von 20 g kommerziell erhältichem Raney Nickel, 2 l Methanol und 15 g Ammoniak bei einer Temperatur von 120°C und 20 bar (2 MPa) Gesamtdruck hydriert. Nach 5 Stunden wurde eine Ausbeute von 94,4% primäres Amin (Pentylamin) erreicht, wobei auch noch Dipentylamin in 5,2%iger Ausbeute erzeugt wurde.

Wird der Raney Nickel (21,9 g) vor der Hydrierung mit 37,5 g Wasser und 5,8 g 35%iger wässriger Formaldehydlösung eine Stunde bei Raumtemperatur (25°C) unter Stickstoff oder Argon gerührt, danach abdekantiert und dreimal mit 50 ml entionisiertem Wasser nachgewaschen, erreicht man unter Verwendung des so modifizierten Katalysators nach 3 Stunden eine Selektivität von 96,9% (Anteil Dipentylamin 3,2%).

### Beispiel 12

### Aceton als Modifikationsmittel

40 g Pynitril wurden in Gegenwart von 6 g kommerziell erhältlichem Raney Nickel, 1,475 l Methanol und 300 g Ammoniak bei einer Temperatur von 110°C und 40 bar (4 MPa) Gesamtdruck hydriert. Nach 5 Stunden wurde eine Ausbeute von 96,4% primäres Amin erreicht, wobei auch noch sekundäres Amin in 1,9%iger Ausbeute erzeugt wurde.

Wird der Raney Nickel (6 g) vor der Hydrierung mit 14 g Wasser und 0,8 g Aceton eine Stunde unter Stickstoff oder Argon gerührt, danach abdekantiert und dreimal mit je 50 ml entionisiertem Wasser nachgewaschen, erreicht man unter Verwendung des so modifizierten Katalysators nach 5 Stunden eine Selektivität von 97,6% (Anteil sekundäres Amin 1,6%).

## Patentansprüche

1. Verfahren zur Modifikation eines Hydrierungskatalysators vom Typ Raney Nickel, Raney Cobalt, Nickel auf Träger oder Cobalt auf Träger, **dadurch gekennzeichnet, dass** man den Hydrierungskatalysator bei Temperaturen von etwa 0°C bis etwa 120°C mit Kohlenmonoxid, Kohlendioxid, Formaldehyd, einem niederen aliphatischen Aldehyd, einem aromatischen Aldehyd, einem aliphatischen Keton, einem aromatischen Keton, einem gemischt aliphatisch/aromatischen Keton, Glyoxal, Pyruvaldehyd oder Glyoxylsäure als Modifikationsmittel in einem aus Wasser oder einem organischen Lösungsmittel bestehenden flüssigen Dispersionsmittel für die Dauer von etwa 15 Minuten bis etwa 24 Stunden behandelt.

2. Verfahren nach Anspruch 1, **dadurch gekennzeichnet, dass** als Modifikationsmittel Kohlenmonoxid, Formaldehyd oder ein niederer aliphatischer Aldehyd verwendet wird.

3. Verfahren nach Anspruch 1 oder 2, **dadurch gekennzeichnet, dass** der niedere aliphatische Aldehyd Acetaldehyd ist.

4. Verfahren nach Anspruch 1, **dadurch gekennzeichnet, dass** der aromatische Aldehyd Benzaldehyd oder Anisaldehyd ist

5. Verfahren nach Anspruch 1, **dadurch gekennzeichnet, dass** der aliphatische, aromatische oder gemischt aliphatisch/aromatischer Keton Aceton ist.

6. Verfahren nach Anspruch 1 oder 2, **dadurch gekennzeichnet, dass** als Modifikationsmittel Formaldehyd verwendet wird.

7. Verfahren nach einem der Ansprüche 1 bis 6, **dadurch gekennzeichnet, dass** das organische Lösungsmittel ein aliphatischer Kohlenwasserstoff, ein aromatischer Kohlenwasserstoff, ein Alkanol, ein aliphatischer oder cyclischer Ether, oder ein Heteroaromat ist.

8. Verfahren nach Anspruch 7, **dadurch gekennzeichnet, dass** das organische Lösungsmittel Pentan, Hexan, Benzen, Toluen, Methanol, Ethanol, Propanol, Diethylether, Tetrahydrofuran, Dioxan oder Pyridin ist.

9. Verfahren nach einem der Ansprüche 1 bis 8, **dadurch gekennzeichnet, dass** die Menge des eingesetzten Modifikationsmittels im Falle von Kohlenmonoxid 0,5 - 5 Gew.%, vorzugsweise 1 - 2 Gew.%, im Falle von Kohlendioxid 1 - 30 Gew.%, vorzugsweise 5 - 15 Gew.%, im Falle von Formaldehyd, einem niederen aliphatischen Aldehyd, einem aromatischen Aldehyd, Glyoxal, Pyruvaldehyd oder Glyoxylsäure 0,25 - 50 Gew.%, vorzugsweise 1 - 15 Gew.%, bzw. im Falle von einem aliphatischen, aromatischen oder gemischt aliphatisch/aromatischen Keton 0,25 - 50 Gew.%, vorzugsweise 5 - 20 Gew.%, jeweils bezogen auf die Menge des eingesetzten zu modifizierenden Hydrierungskatalysators, beträgt.

10. Verfahren nach einem der Ansprüche 1 bis 9, **dadurch gekennzeichnet, dass** das Modifikationsverfahren bei Raumtemperatur durchgeführt wird.

11. Verfahren zur katalytischen Hydrierung eines Nitrils zum entsprechenden primären Amin, **dadurch gekennzeichnet, dass** man als Hydrierungskatalysator einen verwendet, welcher gemäss dem in einem der Anspüche 1 bis 10 definierten Modifikationsverfahren erhalten wurde.

## Claims

1. A process for the modification of a hydrogenation catalyst of the Raney nickel, Raney cobalt, nickel-on-carrier or cobalt-on-carrier type, which process comprises treating the hydrogenation catalyst at temperatures of about 0°C to about 120°C with carbon monoxide, carbon dioxide, formaldehyde, a lower aliphatic aldehyde, an aromatic aldehyde, an aliphatic ketone, an aromatic ketone, a mixed aliphatic/aromatic ketone, glyoxal, pyruvaldehyde or glyoxylic acid as the modification agent in a liquid dispersion medium consisting of water or an organic solvent for a duration of about 15 minutes to about 24 hours.

2. A process according to claim 1, wherein carbon monoxide, formaldehyde or a lower aliphatic aldehyde is used as the modification agent.

3. A process according to claim 1 or claim 2, wherein the lower aliphatic aldehyde is acetaldehyde.

4. A process according to claim 1, wherein the aromatic aldehyde is benzaldehyde or anisaldehyde.

5. A process according to claim 1, wherein the aliphatic, aromatic or mixed aliphatic/aromatic ketone is acetone.

6. A process according to claim 1 or claim 2, wherein formaldehyde is used as the modification agent.

7. A process according to any one of claims 1 to 6, wherein the organic solvent is an aliphatic hydrocarbon, an aromatic hydrocarbon, an alkanol, an aliphatic or cyclic ether or a heteroaromatic.

8. A process according to claim 7, wherein the organic solvent is pentane, hexane, benzene, toluene, methanol, ethanol, propanol, diethyl ether, tetrahydrofuran, dioxan or pyridine.

9. A process according to any one of claims 1 to 8, wherein the amount of modification agent which is employed amounts to about 0.5 - 5 wt.%, preferably 1 - 2 wt.%, in the case of carbon monoxide; 1 - 30 wt.%, preferably 5 - 15 wt.%, in the case of carbon dioxide; 0.25 - 50 wt.%, preferably 1- 15 wt.%, in the case of formaldehyde, a lower aliphatic aldehyde, glyoxal, pyruvaldehyde or glyoxylic acid; and 0.25 - 50 wt.%, preferably 5 - 20 wt.%, in the case of an aliphatic, aromatic or mixed aliphatic/aromatic ketone, in each case based on the amount of modified hydrogenation catalyst to be employed.

10. A process according to any one of claims 1 to 9, wherein the modification process is carried out at room temperature.

11. A process for the catalytic hydrogenation of a nitrile to the corresponding primary amine, which process comprises using as the hydrogenation catalyst one which has been obtained in accordance with the modification process defined in any one of claims 1 to 10.

## Revendications

1. Procédé pour la modification d'un catalyseur d'hydrogénation de type au nickel de Raney, au cobalt de Raney, au nickel sur support ou au cobalt sur support, **caractérisé en ce que** le catalyseur d'hydrogénation est traité au monoxyde de carbone, au dioxyde de carbone, au formaldéhyde, à un aldéhyde aliphatique bas, à un aldéhyde aromatique, à une cétone aliphatique, à une cétone aromatique, à un mélange de cétone aliphatique/aromatique, au glyoxal, au pyruvaldéhyde ou à l'acide glyoxylique comme agent de modification, dans un milieu de dispersion liquide constitué d'eau ou d'un solvant organique, pour une durée d'environ 15 minutes jusqu'à environ 24 heures, à des températures allant de 0°C jusqu'à 120°C.

2. Procédé selon la revendication 1 **caractérisé en ce que** du monoxyde de carbone, du formaldéhyde ou un aldéhyde aliphatique bas est utilisé comme agent de modification.

3. Procédé selon la revendication 1 ou 2 **caractérisé en ce que** de l'acétaldéhyde est utilisé comme aldéhyde aliphatique bas.

4. Procédé selon la revendication 1 **caractérisé en ce que** du benzaldéhyde ou de l'anisaldéhyde est utilisé comme aldéhyde aromatique.

5. Procédé selon la revendication 1 **caractérisé en ce que** de l'acétone est utilisée comme cétone aliphatique, cétone aromatique ou mélange de cétone mélangée aliphatique/aromatique.

6. Procédé selon la revendication 1 ou 2 **caractérisé en ce que** du formaldéhyde est utilisé comme agent de modification.

7. Procédé selon l'une quelconque des revendications 1 à 6 **caractérisé en ce que** le solvant organique est constitué d'un hydrocarbure aliphatique, d'un hydrocarbure aromatique, d'un alkanol, d'un éther aliphatique ou cyclique ou d'une hétéro-aromatique.

8. Procédé selon la revendication 7 **caractérisé en ce que** le solvant organique est constitué de pentane, de hexane, de benzène, de toluène, de méthanol, d'éthanol, de propanol, de diéthyléther, de tétrahydrofuranne, de dioxanne ou de pyridine.

9. Procédé selon l'une quelconque des revendications 1 à 8 **caractérisé en ce que** la quantité d'agent de modification utilisé est de 0,5 % à 5 % en poids, de préférence 1 à 2 % en poids, dans le cas du monoxyde de carbone ; de 1 à 30 % en poids, de préférence 5 à 15 % en poids, dans le cas du dioxyde de carbone ; de 0,25 à 50 % en poids, de préférence de 1 à 15 % en poids, dans le cas du formaldéhyde, d'un aldéhyde aliphatique bas, d'un aldéhyde aromatique, du glyoxal, de pyruvaldéhyde ou d'acide glyoxylique ; ou de 0,25 à 50 % en poids, de préférence 5 à 20 % en poids, dans le cas d'une cétone aliphatique, aromatique, ou d'un mélange de cétone aliphatique/ aromatique proportions exprimées par rapport à la quantité de catalyseur d'hydrogénation à modifier.

10. Procédé selon l'une quelconque des revendications 1 à 9 **caractérisé en ce que** le procédé de modification est exécuté à la température ambiante.

11. Procédé pour l'hydrogénation catalytique d'un nitrile pour obtenir l'amine primaire correspondante, **caractérisé en ce qu'**on utilise comme catalyseur d'hydro-génation un catalyseur d'hydrogénation obtenu selon le procédé de modification défini dans l'une quelconque des revendications 1 à 10.
